# EUROPEAN PATENT APPLICATION

(11) **EP 2 215 962 A1**
(43) Date of publication of application: **11.08.2010**
(21) Application number: 10152868.5
(22) Date of filing: 09.02.2010
(51) Int. Cl.: A61B 1/005, A61M 25/00, B29C 47/02

(54) **Method for production of flexible tube for endoscope**

(30) Priority: 09.02.2009 JP 2009027064
(71) Applicant: FUJIFILM Corporation, Tokyo (JP)
(72) Inventor: Yago, Atsushi, Saitama-shi Saitama (JP); Miyasaka, Rei, Fujinomiya-shi Shizuoka (JP)
(74) Representative: Klunker . Schmitt-Nilson . Hirsch

(57) **Abstract**

Upon starting continuous extrusion, the temperature of a hard resin (32) and a soft resin (31) is increased in extrusion sections (20, 19). A screw (20a) of the extrusion section of the hard resin is set at high rpm at first. The hard resin reaches a maximum temperature α(°C) by heating of friction with the screw, and comes to have high flowability. Upon starting conveyance of an assembly (28), a large amount of molten hard resin and a small amount of molten soft resin are extruded onto the periphery of a tubular structure (14). Upon passage of a point A of the tubular structure under annular discharge throats (30a, 29a), the extrusion amount of the hard resin starts gradually decreasing, and the extrusion amount of the soft resin starts gradually increasing. Since the temperature of the hard resin starts dropping a short time after the decrease in the extrusion amount, the hard resin maintains the high flowability.

## Description

### BACKGROUND OF THE INVENTION

### 1. Field of the Invention

The present invention relates to a method for production of a flexible tube used in an insert section of an endoscope.

### 2. Description Related to the Prior Art

An endoscope is known as medical equipment for imaging the inside of a living body cavity without incision. The endoscope has an insert section introduced into the human body cavity and a handling section provided on a proximal end of the insert section. The insert section is provided with a slender flexible tube having a diameter of approximately 2 to 15 millimeters and a length of some tenths of a meter to 2 meters.

The flexible tube is constituted of a flexible tubular structure and a covering layer for covering the periphery of the tubular structure. The tubular structure is constituted of a helical coil, which is made of a metal ribbon wound helically, and a tubular net for surrounding the helical coil. The covering layer is made of thermoplastic resin such as a urethane resin, and formed on the periphery of the tubular structure by extrusion.

The flexible tube is expected to be soft at a distal end for better insertability into the body cavity, and hard at a proximal end for better operatability of the handling section. Thus, the covering layer is made of soft resin and hard resin, and varying the ratio between the soft resin and the hard resin allows adjustment of the hardness of the flexible tube. According to Laid-Open Japanese Utility Model Publication No. 55-112505, the thickness of a hard resin layer is decreased with approaching a distal end, though the outside diameter of a covering layer is constant throughout the whole length. According to Japanese Patent Laid-Open Publication Nos. 2-131738 and 3-141920, a covering layer is made of a mixture of soft resin and hard resin. Varying a mixing ratio allows variety in the flexibility of a flexible tube between a distal end side and a proximal end side.

By the way, the hard resin is originally hard, and thus is more difficult to extrude than the soft resin. Therefore, an extrusion amount of the hard resin tends to be uneven.

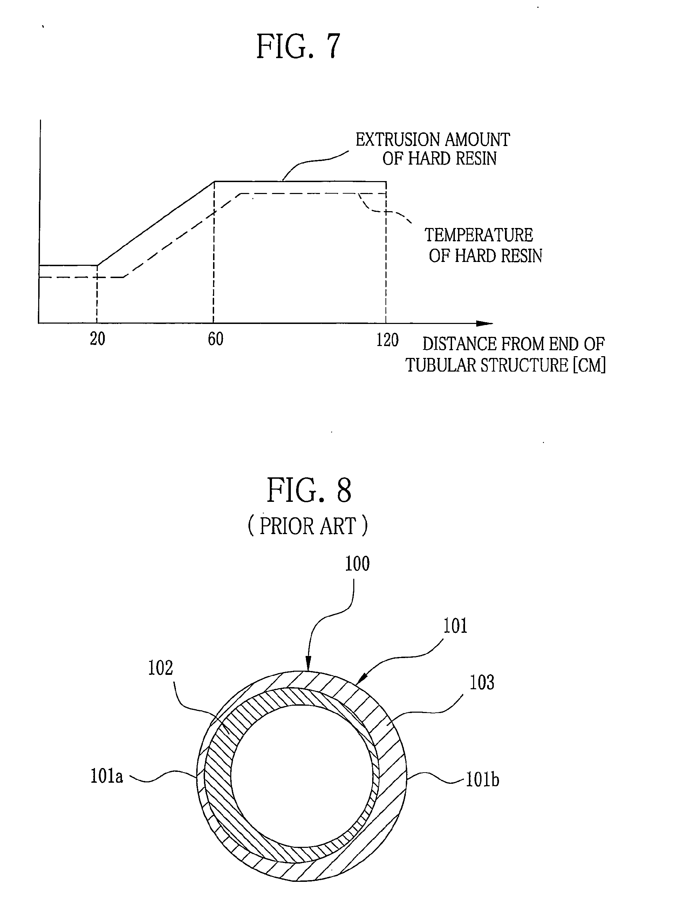

If unevenness in the extrusion amount of the hard resin occurs in a circumferential direction of the flexible tube, as shown in Fig. 8, the thickness of a hard resin layer 102 becomes uneven in the circumference of a flexible tube 100. In this case, a part 101a of the covering layer 101 is hard because of having more hard resin 102 than soft resin 103, whereas another part 101b is soft because of having more soft resin 103 than hard resin 102. As a result, the flexibility of the flexible tube 100 varies depending on a bending direction even in the same device.

If unevenness in the extrusion amount of the hard resin occurs in a longitudinal direction of the flexible tube, the hardness of the covering layer varies discontinuously. If heavy stress is applied to a part whose hardness abruptly varies by twisting operation and the like, the covering layer easily breaks, kinks, or cracks at that part.

### SUMMARY OF THE INVENTION

An object of the present invention is to provide a method for production of a flexible tube for an endoscope by which unevenness in an extrusion amount of hard resin is reduced.

To achieve the above and other objects, in a method according to the present invention, a covering layer for covering a tubular structure is formed from a side of a first end, which contains more hard resin than soft resin, to a side of a second end, which contains more soft resin than hard resin, while the tubular structure passes through a head section of an extrusion apparatus. The covering layer is made of two layers of the hard resin and the soft resin or a mixture of the hard resin and the soft resin. One of the hard resin and the soft resin is gradually decreased in a midway between the first end and the second end, and the other one of the hard resin and the soft resin is gradually increased therein.

The second end is positioned on a side of a distal end of an insert section of an endoscope, and the first end is coupled to a handling section. The extrusion apparatus has a first extrusion section and a second extrusion section. Each of the first extrusion section and the second extrusion section has a screw. The first extrusion section feeds the hard resin into the head section, and the second extrusion section feeds the soft resin into the head section. The screw of the first extrusion section rotates at high speed while extruding a large amount of hard resin, and results in increase in the temperature of the hard resin. Since the hard resin is at high temperature while the extrusion amount of the hard resin is large, the flowability of the hard resin is increased and thickness variations are prevented.

In a preferred embodiment of the present invention, the extrusion apparatus forms a hard resin layer on the periphery of the tubular structure, and then forms a soft resin layer on the hard resin layer. The tubular structure includes a helical coil made of a metal ribbon wound helically and a tubular net for surrounding the helical coil.

According to the present invention, the covering layer is formed from the side of the first end to the side of the second end. On the side of the first end, the screw rotates at high rpm to extrude the large amount of hard resin, and friction with the screw causes increase in the temperature of the hard resin. Although the hard resin has lower flowability than the soft resin, in general, the high temperature of the hard resin allows to prevent undesirable variations in the extrusion amount during the extrusion of the covering layer on the side of the first end. Since the extrusion amount of the hard resin is decreased with approaching the second end, the temperature of the hard resin drops too. The temperature of the hard resin, however, starts dropping a short time after the decrease in the extrusion amount, and hence the flowability of the hard resin is not abruptly reduced even with the gradual decrease in the extrusion amount of the hard resin. Therefore, it is possible to form the covering layer without undesirable extrusion variations throughout the whole length from the first end to the second end.

### BRIEF DESCRIPTION OF THE DRAWINGS

For more complete understanding of the present invention, and the advantage thereof, reference is now made to the following descriptions taken in conjunction with the accompanying drawings, in which:
Fig. 1 is a schematic view of an electronic endoscope;
Fig. 2 is a cross sectional view of a flexible tube;
Fig. 3 is a block diagram showing the schematic structure of a continuous extrusion apparatus;
Fig. 4 is an explanatory view that schematically shows variations in the thicknesses of a hard resin layer and a soft resin layer in extruding a covering layer on an assembly;
Fig. 5 is a graph showing the relation between distance from a proximal end of a tubular structure and the extrusion amount of hard resin, and a graph showing the relation between the distance from the proximal end of the tubular structure and the temperature of the hard resin;
Fig. 6 is a flowchart of a covering layer extrusion process;
Fig. 7 is a graph showing the relation between distance from a distal end of the tubular structure and the extrusion amount of the hard resin, and a graph showing the relation between the distance from the distal end and the temperature of the hard resin, in the case of extruding the covering layer from the side of the distal end of the tubular structure; and
Fig. 8 is a cross sectional view of a covering layer manufactured by a conventional method.

### DESCRIPTION OF THE PREFERRED EMBODIMENTS

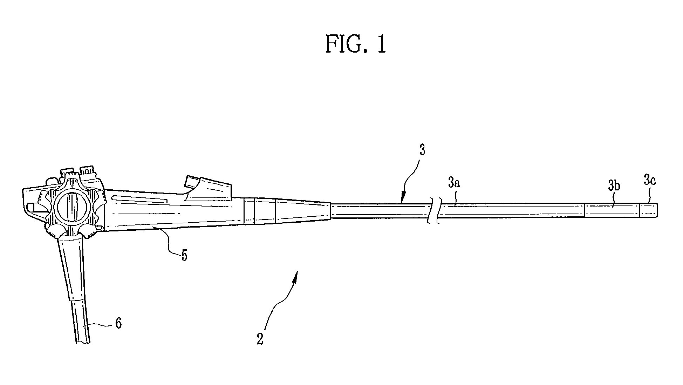

As shown in Fig. 1, an electronic endoscope 2 is constituted of an insert section 3 to be introduced into a human body cavity, a handling section 5 coupled to a base end of the insert section 3, and a universal cord 6 connected to a processor unit and a light source unit.

The insert section 3 has a flexible portion 3a, a bending portion 3b, and a distal portion 3c disposed in this order from the side of the base end. The flexible portion 3a occupies most of the insert section 3. The bending portion 3b flexibly bends inside the body cavity, and aims the distal portion 3c at a desired direction. The distal portion 3c contains an image sensor (not illustrated) for imaging an internal body part.

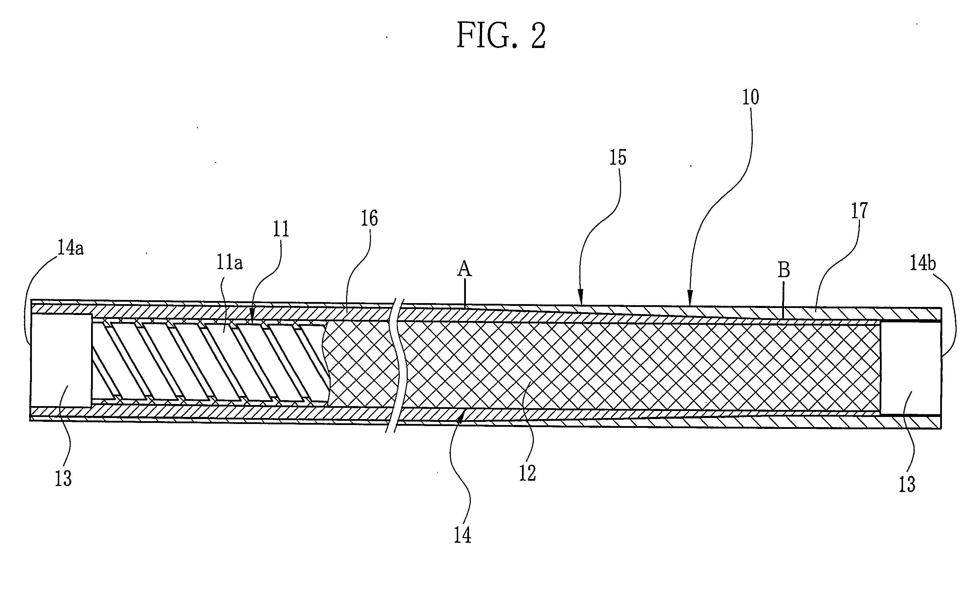

The flexible portion 3a is constituted of a flexible tube 10 shown in Fig. 2. The flexible tube 10 includes a flexible tubular structure 14, a covering layer 15 for covering the periphery of the tubular structure 14 for encapsulation. The tubular structure 14 is constituted of a helical coil 11, a tubular net 12 for covering the periphery of the helical coil 11, and metal rings 13 fitted into both ends of the helical coil 11 and the tubular net 12. The helical coil 11 is made of a metal ribbon 11a wound helically into a tubular shape. The tubular net 12 is made of thin metal wires braided into a tubular shape. The covering layer 15 is made of urethane resin or the like.

The covering layer 15 has a hard resin layer 16 formed on the periphery of the tubular structure 14, and a soft resin layer 17 formed on the periphery of the hard resin layer 16. At a proximal end 14a of the tubular structure 14, which is coupled to the handling section 5, the hard resin layer 16 is thicker than the soft resin layer 17. The thickness of the hard resin layer 16 is gradually reduced from the side of the proximal end 14a to the side of a distal end 14b, and becomes smaller than the soft resin layer 17 at the distal end 14b. Accordingly, the flexible tube 10 has low flexibility (is hard) on the side of the proximal end 14a, and has high flexibility (is soft) on the side of the distal end 14b.

If the total length of the tubular structure 14 is 120 cm, as an example, a point A is positioned at 60 cm from the proximal end 14a, that is, at the midpoint of the tubular structure 14, and a point B is positioned at 40 cm from the point A toward the distal end 14b. At this time, the covering layer 15 is formed at a constant thickness ratio of, for example, "the hard resin layer 16":"the soft resin layer 17"=8:2 in a midway between the proximal end 14a and the point A. The thickness of the hard resin layer 16 is gradually reduced between the point A and the point B. The hard resin layer 16 is thinner than the soft resin layer 17 between the point B and the distal end 14b.

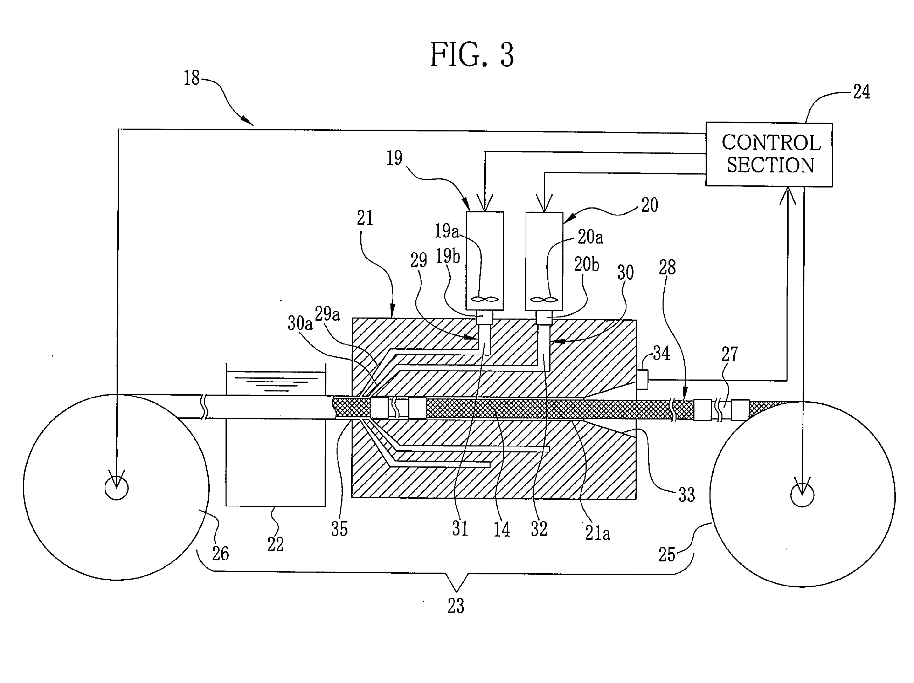

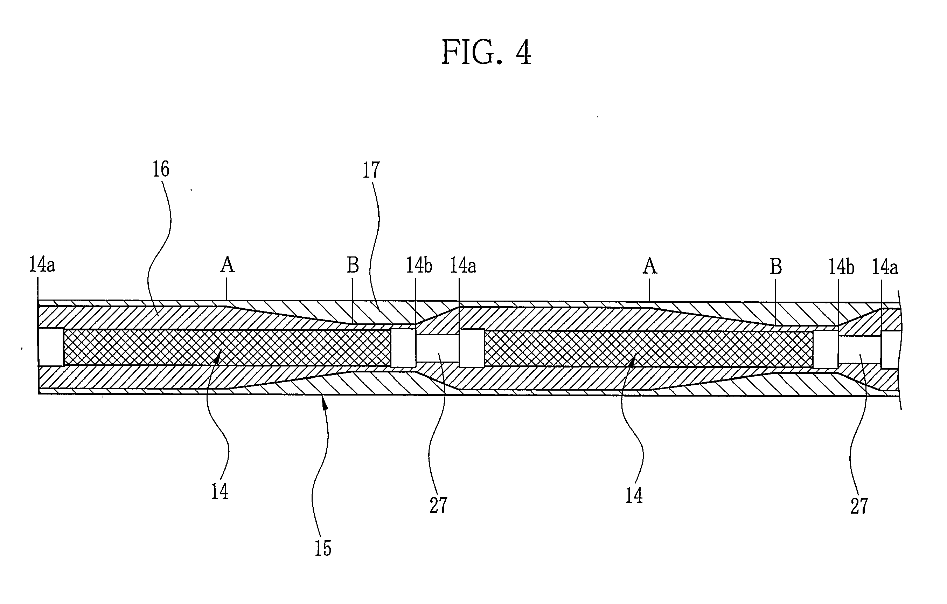

In a covering layer extrusion process, as shown in Figs. 3 and 4, a hard resin and a soft resin are extruded onto the periphery of an assembly 28 into which a plurality of tubular structures 14 are coupled in series with joints 27. As shown in Fig. 3, a continuous extrusion apparatus 18 for forming the covering layer 15 is provided with commonly known extrusion sections 19 and 20, a head section 21, a cooling section 22, an assembly feeding section 23, and a control section 24. Each extrusion section 19 or 20 includes a hopper, a screw 19a or 20a, and the like. In the head section 21, the molten resins from the extrusion sections 19 and 20 are extruded on the periphery of the assembly 28. In the cooling section 22, the extruded resins are cooled to form the covering layer 15. The assembly feeding section 23 feeds the assembly 28 into the head section 21 and the cooling section 22. The control section 24 controls the whole continuous extrusion apparatus 18.

The assembly feeding section 23 has a feed drum 25 and a winding drum 26. The assembly 28 is wound on the feed drum 25 in advance. The assembly 28 is successively pulled out of the feed drum 25 with the proximal end 14a of the tubular structure 14 in the lead. The assembly 28 travels through a circular hole 21a of the head section 21 and the cooling section 22, and is linearly wound up by the winding drum 26. The rotation speeds of the feed drum 25 and the winding drum 26 are controlled by the control section 24, to adjust the conveyance speed of the assembly 28.

A nozzle 19b of the extrusion section 19 is coupled to a soft resin path 29, and a nozzle 20b of the extrusion section 20 is coupled to a hard resin path 30. A molten soft resin 31 is extruded through the soft resin path 29 from an annular discharge throat 29a into the circular hole 21a of the head section 21, and a molten hard resin 32 is extruded through the hard resin path 30 from an annular discharge throat 30a thereinto. The annular discharge throats 29a and 30a are adjacently disposed to each other. The control section 24 regulates the RPMs of the screws 19a and 20a to adjust the extrusion amounts of the molten soft resin 31 and the molten hard resin 32 from the extrusion sections 19 and 20. The temperatures of the hard resin 31 and the soft resin 32 are increased by heating the extrusion sections 19 and 20 and the head section 21. In addition, the higher the RPMs of the screws 19a and 20a, the higher the temperatures of the soft resin 31 and the hard resin 32 become, and resulting in increase in flowability.

To regulate the thicknesses of the hard resin layer 16 and the soft resin layer 17, the conveyance speed of the assembly 28 is kept constant, and the extrusion amounts of the molten soft resin 31 and the molten hard resin 32 are varied. A conical recess 33 is formed in the head section 21 to guide insertion of the assembly 28 into the circular hole 21a. A sensor 34 for detecting the joint 27 is provided on the head section 21 in the vicinity of an entrance of the conical recess 33.

The annular discharge throat 29a of the soft resin path 29 is positioned downstream of the conveyance direction of the assembly 28, and the annular discharge throat 30a of the hard resin path 30 is positioned upstream thereof. Since the molten soft resin 31 and the molten hard resin 32 are simultaneously extruded from the annular discharge throats 29a and 30a into the circular hole 21a of the head section 21, respectively, the molten hard resin 32 from the hard resin path 30 is first applied to the assembly 28, prior to the application of the molten soft resin 31 from the soft resin path 29. Consequently, the hard resin layer 16 is formed below, and the soft resin layer 17 is formed above.

The inside diameter of an exit 35 of the circular hole 21a is formed so as to coincide with the outside diameter of the covering layer 15. Since the assembly 28 goes out of the exit 35 immediately after the extrusion of the molten hard resin 32 and the molten soft resin 31, the outside diameter of the covering layer 15 is made constant throughout its length.

The assembly 28 that has passed through the head section 21 is led to the cooling section 22. In the cooling section 22, there is a tank with coolant such as water. While the assembly 28 travels through the coolant, the hard resin 32 and the soft resin 31 are hardened and become the covering layer 15. Instead of above, coolant, air or the like may be blown to the assembly 28 to cool the resins 32 and 31.

The covering layer extrusion process with use of the continuous extrusion apparatus 18 will be described with referring to Figs. 4 to 6. Fig. 4 schematically shows variation in the thicknesses of the hard resin layer 16 and the soft resin layer 17, and shows the covering layer 15 thicker than it really is for the sake of visual clarity. The covering layer 15 is formed from the left side to the right side of Fig. 4.

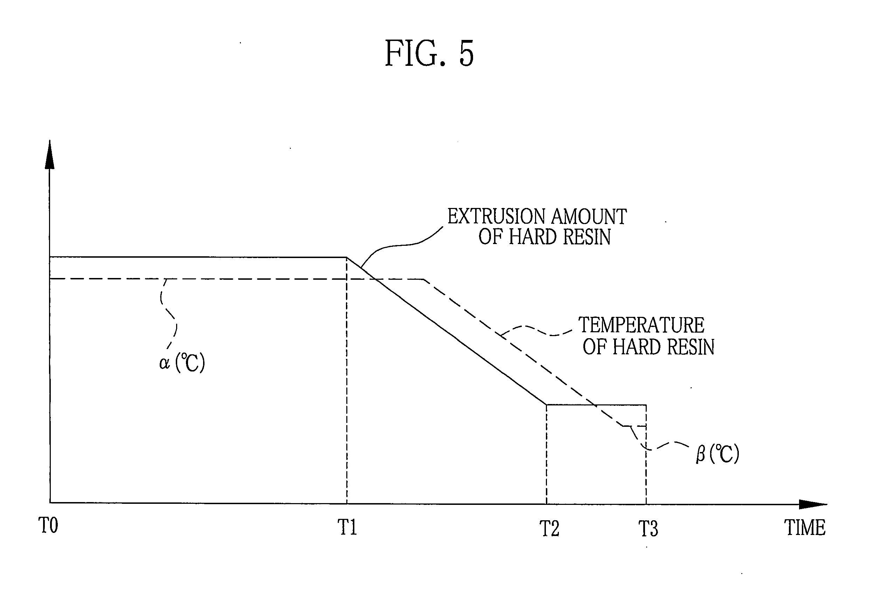

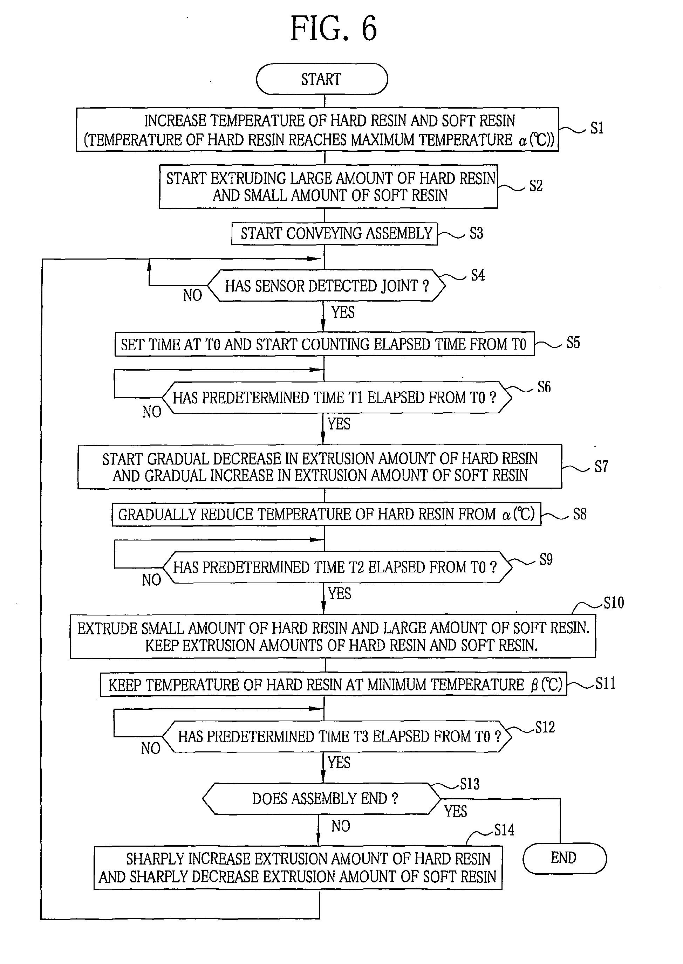

Referring to Fig. 6, after the setting of the assembly 28 on the continuous extrusion apparatus 18, upon starting continuous extrusion, the hard resin 32 and the soft resin 31 are heated in the extrusion sections 19 and 20. At this time, the screw 20a of the extrusion section 20 is set at high RPM for a large extrusion amount. Thus, the temperature of the hard resin layer 32 reaches a maximum temperature α(°C) (refer to Fig. 5) by addition of friction heat with the screw 20a, and the hard resin 32 has high flowability (step 1).

The extrusion section 20 extrudes the molten hard resin 32 from the annular discharge throat 30a of the hard resin path 30 on the periphery of the assembly 28, and the extrusion section 19 extrudes the molten soft resin 31 from the annular discharge throat 29a of the soft resin path 29 on the hard resin 32. At this time, the extrusion amount of the molten hard resin 32 is large and the extrusion amount of the molten soft resin 31 is small, so that the hard resin layer 16 and the soft resin layer 17 have a thickness ratio of eight to two (step 2).

Then, conveyance of the assembly 28 is started (step 3). During the conveyance of the assembly 28, the extrusion section 20 keeps extruding the molten hard resin 32 with the screw 20a at the high RPM, so that the hard resin 32 maintains the high flowability. Therefore, the extrusion amount of the hard resin 32 does not have undesirable variations.

If the sensor 34 detects the joint 27 (step 4), the control section 24 actuates a timer (not illustrated) to measure a lapse of time from a time T0 of the detection (step 5). When a measurement coincides with a predetermined time T1 (step 6), the point A of the tubular structure 14 is positioned right below the annular discharge throats 29a and 30a. Then, the control section 24 controls the extrusion section 20 so as to gradually decrease the extrusion amount of the molten hard resin 32, and controls the extrusion section 19 so as to gradually increase the extrusion amount of the molten soft resin 31 (step 7).

Although the annular discharge throats 29a and 30a are adjacent to each other, the annular discharge throats 29a and 30a are not placed in the same position. Thus, the point A cannot be positioned simultaneously right below both of the annular discharge throats 29a and 30a in the strict sense. However, it is described that the point A is positioned right below the annular discharge throats 29a and 30a after a lapse of the predetermined time T1 from the time T0, for the sake of brevity of description. The same goes for the point B and the proximal end 14b.

While the extrusion amount of the molten hard resin 32 is gradually decreased, the RPM of the screw 20a is gradually reduced, and consequently the temperature of the hard resin 32 gradually drops from the maximum temperature α(°C). The temperature of the hard resin 32, as shown by a broken line of Fig. 5, does not drop immediately but starts dropping a short time after the gradual decrease in the extrusion amount (step 8). Accordingly, the hard resin 32 still has the high flowability, and the extrusion amount of the hard resin 32 is gradually decreased without undesirable variations. Since the soft resin 31 originally has high flowability, the extrusion amount of the soft resin 31 does not undesirably vary.

When a predetermined time T2 has elapsed from the time T0 (step 9), the assembly 28 is further conveyed at 40 cm after the point A of the tubular structure 14 has passed under the annular discharge throats 29a and 30a, and the point B of the tubular structure 14 is positioned right below the annular discharge throats 29a and 30a. At the point B, the extrusion amount of the hard resin 32 is smaller than that of the soft resin 31. The extrusion amounts of the hard resin 32 and the soft resin 31 are maintained from then on (step 10). The screw 20a is kept constant at low RPM. A short time after the point B of the tubular structure 14 is positioned right below the annular discharge throats 29a and 30a, the temperature of the hard resin layer 32 reaches a minimum temperature β(°C), and is kept there (refer to Fig. 5) (step 11).

When a predetermined time T3 has elapsed from the time T0 (step 12), the assembly 28 is further conveyed at 20 cm after the point B of the tubular structure 14 has passed under the annular discharge throats 29a and 30a, and the distal end 14b of the tubular structure 14 is positioned right below the annular discharge throats 29a and 30a. If the distal end 14b of the tubular structure 14 is an end of the assembly 28 (YES in step 13), the covering layer extrusion process is completed. However, if the end 14b of the tubular structure 14 is coupled to another tubular structure 14 with the joint 27, the control section 24 switches the screw 20a from the low RPM to the high RPM, and the extrusion amount of the hard resin 32 is sharply increased. At the same time, the screw 19a is switched from high RPM to low RPM, and the extrusion amount of the soft resin 31 is sharply decreased (step 14).

Then, the operation returns to the step 4. The steps 4 to 13 are repeated until the sensor 34 does not detect the joint 27 and the end of the assembly 28 passes below the annular discharge throats 29a and 30a (YES in step 13).

Since the assembly 28 goes out of the exit 35 immediately after the extraction of the molten hard resin 32 and the molten soft resin 31, the outside diameter of the flexible tube 10 is made constant. After that, while the assembly 28 travels through the cooling section 22, the hard resin 32 and the soft resin 31 are cooled and hardened into the covering layer 15.

The assembly 28 on which the covering layer 15 is formed throughout the whole length is detached from the continuous extrusion apparatus 18. Then, the assembly 28 is cut into the plurality of flexible tubes 10, and the joints 27 are detached.

In the covering layer 15 of the flexible tube 10, since the hard resin layer 16 and the soft resin layer 17 are extruded without undesirable variations, the flexible tube 10 has constant flexibility and constant hardness in a circumferential direction regardless of a bending direction. Also, the hardness of the flexible tube 10 continuously varies in a longitudinal direction, and hence the covering layer 15 resists breaking, kinking, or cracking even with twisting operation.

The flexible tube 10 manufactured as described above constitutes the flexible portion 3a. A soft end (distal end 14b) of the flexible tube 10 is coupled to the bending portion 3b, and a hard end (proximal end 14a) thereof is coupled to the handling section 5. Accordingly, the insert section 3 has high flexibility at a distal end side for better insertability into the body cavity, whereas has low flexibility at a proximal end side for better operatability.

If the covering layer 15 is formed on the tubular structure 14 from the side of the distal end 14b, as shown in Fig. 7, the extrusion of the molten hard resin 32 is started while the temperature of the hard resin 32 stays at low. While the extrusion amount of the hard resin 32 is gradually increased, increase in the temperature of the hard resin 32 cannot keep up with increase in the extrusion amount of the hard resin 32. As a result, since the hard resin 32 is extruded with low flowability, the hard resin layer 16 tends to have undesirable variations.

In the above embodiment, the covering layer 15 consists of the hard resin layer 16 and the soft resin layer 17. Otherwise, a mixture of the molten soft resin 31 and the molten hard resin 32 may be extruded onto the periphery of the assembly 28 from a single annular discharge throat by mixing extrusion. In this case, the annular discharge throats 29a and 30a are integrated. The molten soft resin 31 and the molten hard resin 32 are mixed, and then extruded onto the periphery of the assembly 28.

The present invention is applicable to other types of endoscopes having a flexible tube, in addition to the electronic endoscope.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A method for production of a flexible tube (10) for an endoscope (2), the flexible tube including a tubular structure (14) and a covering layer (15) of a constant thickness formed on a periphery of the tubular structure, the covering layer being made of two layers of a hard resin (32) and a soft resin (31) or a mixture of the hard resin and the soft resin, the covering layer containing more of the hard resin than the soft resin on a side of a first end (14a) and more of the soft resin than the hard resin on a side of a second end (14b), one of the hard resin and the soft resin gradually decreasing in a midway between the first end and the second end and the other one of the hard resin and the soft resin gradually increasing therein, the method comprising the steps of:
passing the tubular structure through a head section (21) of an extrusion apparatus (18) by advancing the first end; and
extruding the hard resin and the soft resin while the tubular structure passes through the head section to form the covering layer from the side of the first end.

2. The method according to claim 1, wherein the second end (14b) is positioned on a side of a distal end of an insert section (3) of the endoscope (2).

3. The method according to claim 1, wherein the extrusion apparatus (18) has a first extrusion section (20) and a second extrusion section (19), each of the first extrusion section and the second extrusion section has a screw (20a, 19a), the first extrusion section feeds the hard resin (32) into the head section (21), and the second extrusion section feeds the soft resin (31) into the head section.

4. The method according to claim 3, wherein the screw (20a) of the first extrusion section (20) rotates at high speed while extruding a large amount of hard resin (32) to increase a temperature of the hard resin.

5. The method according to claim 4, wherein in the extrusion apparatus (18), a layer of the hard resin (32) is formed on a periphery of the tubular structure (14), and then a layer of the soft resin (31) is formed on the hard resin.

6. The method according to claim 5, wherein the tubular structure (14) comprises:
a helical coil (11) made of a metal ribbon wound helically; and
a tubular net (12) for surrounding the helical coil.
